(19) Europäisches Patentamt
European Patent Office
Office européen des brevets

(11) **EP 3 260 120 B1**

(12) **EUROPEAN PATENT SPECIFICATION**

(45) Date of publication and mention
of the grant of the patent:
**16.12.2020 Bulletin 2020/51**

(51) Int Cl.:
**A61K 31/4439** *(2006.01)*     **A61K 9/127** *(2006.01)*
**A61P 35/00** *(2006.01)*

(21) Application number: **17177183.5**

(22) Date of filing: **21.06.2017**

(54) **PHARMACOLOGICAL ADJUVANTS FOR THERMAL ABLATION OF CANCER**

PHARMAKOLOGISCHE ADJUVANZIEN ZUR THERMISCHEN ABLATION VON KREBS

ADJUVANTS PHARMACOLOGIQUES POUR ABLATION THERMIQUE D'UN CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2016 IT UA20164630**

(43) Date of publication of application:
**27.12.2017 Bulletin 2017/52**

(73) Proprietors:
- **Blasi, Paolo**
  **63838 Belmonte Piceno (FM) (IT)**
- **Calvitti, Mario**
  **06127 Perugia (IT)**
- **Giovagnoli, Stefano**
  **06124 Perugia (IT)**
- **Lilli, Cinzia**
  **06132 Perugia (IT)**
- **Ricci, Maurizio**
  **06123 Perugia (IT)**
- **Rossi, Carlo**
  **06126 Perugia (IT)**
- **Schoubben, Aurelie Marie-Madeleine Denise**
  **06134 Perugia (IT)**
- **Vedovelli, Angelo**
  **06081 Assisi (PG) (IT)**

(72) Inventors:
- **Blasi, Paolo**
  **63838 Belmonte Piceno (FM) (IT)**
- **Calvitti, Mario**
  **06127 Perugia (IT)**
- **Giovagnoli, Stefano**
  **06124 Perugia (IT)**
- **Lilli, Cinzia**
  **06132 Perugia (IT)**

- **Ricci, Maurizio**
  **06123 Perugia (IT)**
- **Rossi, Carlo**
  **06126 Perugia (IT)**
- **Schoubben, Aurelie Marie-Madeleine Denise**
  **06134 Perugia (IT)**
- **Vedovelli, Angelo**
  **06081 Assisi (PG) (IT)**

(74) Representative: **Bianchetti Bracco Minoja S.r.l.**
**Via Plinio, 63**
**20129 Milano (IT)**

(56) References cited:
**EP-A1- 2 548 555**     **WO-A1-2017/048860**
**WO-A2-2005/077365**   **CN-A- 104 546 718**
**CN-A- 104 546 718**   **CN-B- 103 127 003**
**US-A1- 2009 227 633**

- OVERHOLT B F ET AL: "Photodynamic therapy for Barrett's esophagus: follow-up in 100 patients", GASTROINTESTINAL ENDOSCOPY, ELSEVIER, NL, vol. 49, no. 1, 1 January 1999 (1999-01-01), pages 1-7, XP027500214, ISSN: 0016-5107, DOI: 10.1016/S0016-5107(99)70437-2 [retrieved on 1999-01-01]
- BOAS F EDWARD ET AL: "Adjuvant Medications That Improve Survival after Locoregional Therapy", JOURNAL OF VASCULAR AND INTERVENTIONAL RADIOLOGY, vol. 28, no. 7, 1999, page 971, XP085094602, ISSN: 1051-0443, DOI: 10.1016/J.JVIR.2017.04.016

EP 3 260 120 B1

- **TACKER J R ET AL: "DELIVERY OF ANTI TUMOR DRUG TO BLADDER CANCER BY USE OF PHASE TRANSITION LIPOSOMES AND HYPER THERMIA", JOURNAL OF UROLOGY, vol. 127, no. 6, 1982, pages 1211-1214, XP002769669, ISSN: 0022-5347**

Remarks:

The file contains technical information submitted after the application was filed and not included in this specification

**Description**

The present invention relates to the use of proton pump inhibitors in thermal ablation of cancer, and in particular to the use of proton pump inhibitors encapsulated in liposomes. Furthermore, the invention relates to proton pump inhibitors in acid solution encapsulated in liposomes coated with polyethylene glycol and to pharmaceutical formulations containing them.

The invention is defined by the claims. Any subject-matter or embodiment disclosed herein and not covered by the claims does not form part of the claimed invention.

**Background of the invention**

Hepatocellular carcinoma (HCC) has become the fifth most frequent tumour and the second leading cause of cancer-related death in the world. The liver is the most common site for metastatic disease, particularly from colorectal cancer. Partial hepatectomy is the treatment of choice for primary and secondary liver cancer (liver-only disease), but 80-90% of patients are not eligible for resection for a variety of reasons that include location or number of lesions, inadequate functional reserve of the organ, or major comorbidities. Percutaneous hyperthermal tumour ablation is a minimally invasive procedure that offers a potentially radical treatment for patients with inoperable primary or secondary liver cancer. In the case of small HCC (<2-3 cm) this procedure is considered the first-line treatment even when the patient is operable. Widely used for liver cancers, thermal ablation is increasingly employed for patients with kidney, lung and bone cancer that is unresectable or for which other forms of treatment have failed.

Thermal ablation is performed with the use of radiofrequency, microwaves or laser energy to generate high temperatures (60-100°C) around an applicator inserted percutaneously in the centre of the tumour using imaging guidance. Another hyperthermal ablation technique with imaging guidance is based on the use of high-intensity focused ultrasound (HIFU) from an extracorporeal source.

The combination of these local therapies with systemic drugs has aroused great interest, with a view to increasing the success of thermal ablation techniques in tumours of medium and large size.

The aim is to extend the necrosis to tumour sites characterized by reversible cell damage due to exposure to sub-lethal temperatures (<50°C), such as peripheral sites in larger tumours and those close to vessels larger than > 3 mm (heat-sink effect). Particular attention has been paid to chemotherapeutics carried in liposomes (Ahmed M, Chem Phys Lipids 165: 424-37, 2012; Chu KF, Nat Rev Cancer 14: 199-208, 2014).

**Doxil**® (Janssen) is a formulation of **doxorubicin** encapsulated in liposomes coated with polyethylene glycol (PEG), long used in clinical oncology. Encapsulation in pegylated liposomes or liposomes stabilized by polysaccharides like dextran (Pain D, J. Biosci 6: 811-6, 1984; Ning S, Soft Matter 7: 9394-401, 2011) may give anticancer drugs, especially doxorubicin, a higher intratumoral concentration and lower distribution to normal tissues than its soluble (free) form, with advantages in terms of therapeutic efficacy and systemic toxicity, because surface modification with PEG or dextran considerably prolongs the circulation time of liposomes in the blood (stealth liposomes), allowing "passive targeting" in solid tumours, while liposomes do not penetrate normal endothelium (typical gaps 5-10 nm) due to their size (~100 nm), and the discontinuous neoangiogenetic endothelium in tumours (gaps up to 600-800 nm) allows extravasation of the pegylated liposomes during their repeated passages in the microvascular tumour bed. This, together with poor lymphatic drainage, promotes accumulation of the liposomes in the interstitial space of tumours (Enhanced Permeation and Retention effect), where they release doxorubicin, which then diffuses as a free drug in the cells.

**ThermoDox**® (Celsion) is a formulation of pegylated thermosensitive liposomes containing doxorubicin, specifically designed as an adjuvant for thermal ablation. Due to the particular composition of the lipid membrane, these liposomes are subject to nanopore formation when exposed to temperatures >39°C, with an extremely rapid release of the doxorubicin content (80% of the drug release in 20 seconds at 41°C, in vitro). Massive intravascular release of doxorubicin at the tumour site, followed by the diffusion of the molecule in the tumour mass, is the characteristic that distinguishes ThermoDox® from Doxil®, in which the non-thermosensitive liposomes undergo extravasation followed by slow spontaneous release of doxorubicin in the tumour interstitium (Koning GA, Pharm Res 27: 1750-4, 2010; Landon CD, Open Nanomed J 3: 38-64, 2011; Manzoor AA, Cancer Res 72: 5566-75, 2012; Kneidl B, Int J Nanomedicine 9: 4387-98, 2014).

Studies based on animal models of cancer (mammary carcinoma or sarcomas) have demonstrated that intravenous administration of Doxil®, ThermoDox® or pegylated liposomes loaded with doxorubicin, or **cisplatin**, or **5-fluorouracil**, or **paclitaxel**, in combination with percutaneous radio frequency ablation, give rise to drug accumulation in the periablation zone, increase the area of necrosis and improve the animal survival endpoint.

However, the adjuvant effect depends on the sensitivity of the solid tumour to the chemotherapeutic agent. For example, administration of liposomal doxorubicin (ThermoDox®) did not improve the clinical outcome of percutaneous thermoablation in a large group of patients with hepatocellular carcinoma, a tumour highly resistant to chemotherapeutic drugs. In this clinical study (HEAT study), ThermoDox® failed the primary endpoint represented by progression-free survival (Lencioni R, Presentation at the European Conference on Interventional Oncology (ECIO), Budapest (Hungary) June 19-22, 2013; Poon RT, Presentation at the World Conference on Interventional Oncology (WCIO), New York (NY) May 16-19, 2013; Finn RS, Presentation at the 8th International Liver Cancer Association (ILCA) Annual Conference, Kyoto (Japan) September 5-7, 2014). Even the secondary overall survival endpoint did not show significant differences. These limitations clearly suggest that the development of a different adjuvant strategy is required.

Due to their abnormal glucose metabolism, cancer cells are obliged to eliminate protons continuously in order to maintain a neutral intracellular pH, which is crucial for cell survival and function. **V-ATPase**, a plasma membrane proton pump that actively transfers $H^+$ either outside the cell or inside intracellular organelles, is one of the principal mechanisms whereby tumour cells avoid intracellular acidification. Since it is well established that reduction of the intracellular pH makes tumour cells more heat-sensitive (Baronzio GF, Hyperthermia in cancer treatment: a primer, Baronzio GF, Hager ED (eds), pp 67-91, Landes-Springer Publishers, Georgetown, 2006; Song CW, Cancer drug resistance Teicher BA (ed), pp 21-42, Humana Press, Totowa, 2006; Jones EL, Principles and practice of radiation oncology 5th ed, Halperin EC, Perez CA, Brady LW (eds), pp 637-68, Lippincott Williams & Wilkins, Philadelphia, 2008; Viglianti BL, Holland-Frei cancer medicine 8th ed, Ki Hong W, Bast RC, Hait WN, et al (eds), pp 528-40, People's Medical Publishing House-USA, Shelton, 2010), **V-ATPase** inhibition could be exploited to enhance thermal killing.

Omeprazole heads a class of proton pump inhibitors (PPIs) that block $H^+/K^+$-ATPase, the proton pump of gastric parietal cells that releases acid into the lumen of the secretory canaliculi. Omeprazole and its congeners (esomeprazole, lansoprazole, pantoprazole and rabeprazole) are widely used in humans to suppress the secretion of gastric acid in acid-related diseases (peptic ulcer, gastroesophageal reflux disease), with minimal side effects even when administered over the long term and at high dosages. Omeprazole and the PPIs of the same class also inhibit **V-ATPase,** although at higher doses than $H^+/K^+$-ATPase.

PPIs are prodrugs that circulate in the blood in an inactive form and accumulate selectively in the secretory canaliculi of gastric parietal cells, where they are converted into active $H^+/K^+$-ATPase inhibitors. Whole-body autoradiography studies with $^{14}$C-radiolabelled omeprazole in mice have demonstrated that 1 minute after intravenous administration, the radio labelled molecule could be detected primarily in the stomach, liver, lungs and kidneys; after 5 minutes it was located mainly in the stomach and liver; after 16 hours it was confined exclusively to the gastric wall, with selective localization in the secretory spaces of the gastric parietal cells, the proton pump site (Helander HF, Scand J Gastroenterol 20 (Suppl 108): 95-104, 1985). The reason behind this particular biodistribution is that PPIs are weak bases with a pKa around 4. Moreover, in the acidic canalicular space of the stimulated parietal cells (pH ~1), they are extensively protonated and poorly diffusible and this determines the confinement. In addition to "ion trapping", protonation in an acidic environment gives rise to a rapid transformation of PPIs into the sulphonamide derivative, which is the active inhibitor of $H^+/K^+$-ATPase. These are the special features that make PPI drugs highly specific for the treatment of diseases caused by gastric acid hypersecretion (Olbe L, Nat Rev Drug Discov 2: 132-9, 2003).

Since the anomalous pH gradient in tumours (intracellular > extracellular) is the result of activation of proton extrusion systems, such as **V-ATPase**, and is also recognized to be responsible for improved cell survival, tumour progression, metastatic invasion and resistance to antineoplastic drugs, omeprazole and the related PPIs have been extensively investigated for their potential impact in the oncology field. In vitro and in vivo preclinical studies in a wide variety of cancer models have demonstrated that these substances, by blocking proton extrusion, acidify the intracellular pH, increase the extracellular pH to neutrality and produce promising anticancer effects that are not only antiproliferative and cytotoxic, but also chemosensitizing (De Milito A, Curr Pharm Des 18: 1395-406, 2012; Spugnini EP, J Exp Clin Cancer Res 29: 44, 2010; Fais S, J Intern Med 267: 515-25, 2010; Parks SK, Nat Rev Cancer 13: 611-23, 2013; Fais S, Cancer Metastasis Rev 33: 1095-108, 2014; Matuszcak C, Can Cell Microenviron 2: e667, 2015; Walsh M, J Exp Clin Cancer Res 34: 93, 2015).

A recent pilot clinical study demonstrated that the combination with PPIs improves the efficacy of the cisplatin + docetaxel regimen in the treatment of patients with metastatic breast cancer (Wang BY, J Exp Clin Cancer Res 34: 85, 2015).

Other clinical trials are under way to verify the effect of PPIs in implementing chemotherapy for cancer patients (https://clinicaltrials.gov/ct2/show/NCT01748500).

**Brief description of Figures**

Figure 1: Size distribution of PEGylated LUVs loaded and not loaded with omeprazole.
Figure 2: TEM images of LUVs loaded with omeprazole in acidic solution: A (90000x), B (180000x).

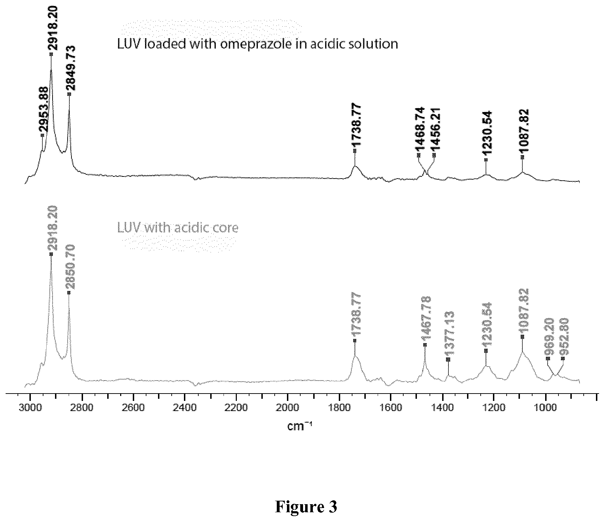

Figure 3: FTIR-ATR profiles of LUVs loaded with omeprazole in acidic solution and LUVs not loaded with the drug (acidic core).

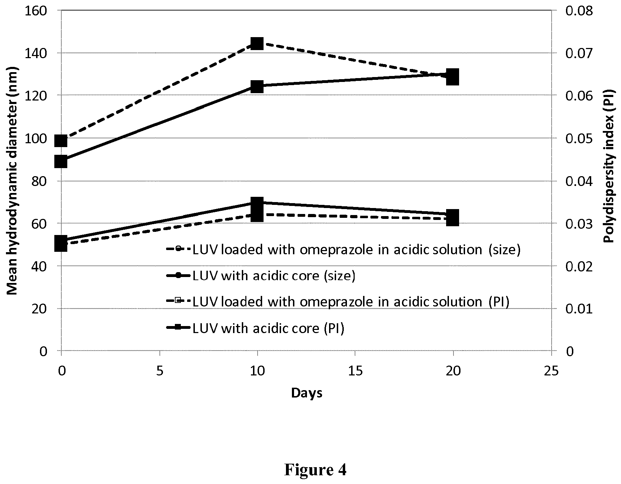

Figure 4: Comparison of the physical stability of LUVs loaded with omeprazole in acidic solution and LUVs not loaded with the drug (acidic core).

Figure 5: Fluorescence microscopy. Effect of drug treatments on the survival of HT-29 cells in culture at 37°C. Staining with ethidium bromide and fluorescein diacetate: living cells (stained in green in the original images) appear as white particles in the left-hand column. Dead cells (stained in red in the original images) appear as white particles in the right-hand column. **A.** Untreated controls; **B.** Omeprazole in acidic solution; **C.** LUVs with acidic core; **D.** LUVs loaded with omeprazole in acidic solution.

Figure 6: Fluorescence microscopy. Effect of drug treatments on the survival of HT-29 cells exposed to 45°C for 2 h. Staining with ethidium bromide and fluorescein diacetate: living cells (stained in green in the original images) appear as white particles in the left-hand column. Dead cells (stained in red in the original images) appear as white particles in the right-hand column. **A.** Untreated controls; **B.** Omeprazole in acidic solution; **C.** LUVs with acidic core; **D.** LUVs loaded with omeprazole in acidic solution.

**Description of the invention**

It has now been found that omeprazole reduces cell survival to heat stress in cancer cell lines in vitro and that liposomal formulations loaded with omeprazole or other PPIs, such as esomeprazole, lansoprazole, pantoprazole and rabeprazole in which the PPIs are encapsulated in a liposome coated with a hydrophilic polymer which is polyethylene glycol (PEG) are particularly active in increasing heat cell damage, and are therefore ideal adjuvants for thermal tumour ablation procedures.

The subject of the present invention is therefore proton pump inhibitor encapsulated in a PEGylated liposome wherein the inhibitor is dissolved in a solution at a pH ranging between 5 and 7.4 for use in the treatment of tumours in combination with a tumour thermoablation method.

The liposome is coated with a hydrophilic polymer which is polyethylene glycol (PEG), which increases its plasma half-life and stability.

The PPI encapsulated in the liposome is dissolved in an acidic solution that, by protonation, causes the direct transformation of the PPI into its biological active form, able to inhibit the proton pump.

Other aspects of the invention are a PPI encapsulated in a liposome coated with polyethylene glycol, and pharmaceutical formulations comprising it.

**Detailed description of the invention**

Object of the present invention is a proton pump inhibitor encapsulated in a PEGylated liposome wherein the inhibitor is dissolved in a solution at a pH ranging between 5 and 7.4 for use in the treatment of tumours in combination with a tumour thermoablation method.

The PPI is preferably selected from omeprazole, esomeprazole, lansoprazole, pantoprazole and rabeprazole. Omeprazole is particularly preferred.

A further object is a proton pump inhibitor encapsulated in a PEGylated liposome wherein the inhibitor is dissolved in a solution at a pH ranging between 5 and 7.4 wherein the proton pump inhibitor is selected from the group consisting of omeprazole, esomeprazole, lansoprazole and pantoprazole.

The PPI can then be encapsulated in a liposome coated with a hydrophilic polymer using known procedures, as described in US5013556, for example.

The process is preferably performed under the conditions necessary to encapsulate the PPI in LUV (*Large Unilamellar Vesicle*) liposomes coated with PEG (PEGylated LUVs), in the presence of a physiologically compatible acid, to obtain an acidic core.

The method comprises dissolving the PPIs and lipids able to form initially multilamellar vesicles (MLV) in an suitable organic solvent or in a mixture of organic solvents.

Typically, the weight ratio between the lipids and the PPI ranges between 99:1 and 80:20, preferably between 99:1 and 90:10.

In a preferred embodiment the lipids employed are hydrogenated soy phosphatidylcholine (HSPC), PEGylated distearoylphosphatidylethanolamine, preferably DSPE-mPEG2000, and cholesterol (Chol), preferably in an HSPC:DSPE-mPEG2000:Chol 60:20:20 (w/w/w) ratio, the inhibitor is omeprazole, and the ratio between lipids and the inhibitor is 95:5. It is preferable to operate in chloroform.

The organic solution obtained is evaporated under vacuum or under an inert gas flux to obtain a lipid film that is hydrated with an aqueous solution of a physiologically compatible acid. Depending on the lipid composition, the lipid film is typically hydrated at a temperature ranging between 50 and 65°C. Preferably, the lipid film is hydrated with a physiologically compatible acid solution at a pH ranging between 5 and 7.4, preferably with lactate buffer at pH 5.0, working at 60°C. The resulting dispersion containing multilamellar vesicles MLV is subjected to a process of particle-size reduction, typically by means of vesicle extrusion through one or more polycarbonate membranes with a pore size ranging between 200 and 100 nm. Typically, the vesicle suspension is recycled through a series of membranes until the desired liposome size is reached. The liposomes can be extruded through a series of membranes with a decreasing pore size to obtain a gradual reduction in the liposome size. The extrusion is conducted at a temperature ranging between 50 and 65°C, depending on the lipid composition, preferably at 60°C. The LUVs obtained are finally ultracentrifuged, and the acidic supernatant containing the free PPI is removed by means of one or more washing cycles and consecutive centrifugation with saline solution.

In cancer cell lines, PPIs and the liposomes according to the invention containing them increase thermal cell damage, and are therefore useful as adjuvants for hyperthermic thermal tumour ablation procedures, in particular for liver, kidney, lung and bone tumours.

It has also now been found that liposomal formulations of omeprazole or other PPIs, such as esomeprazole, lansoprazole, pantoprazole and rabeprazole, in particular formulations where the PPIs are encapsulated in liposomes coated with a hydrophilic polymer which is polyethylene glycol (PEG) are more potent than the corresponding PPI in free form which is not encapsulated in liposomes in reducing cancer cell survival even at 37°C, ie. under normothermic conditions.

In combination with the hyperthermic thermal tumour ablation procedure, PPIs may be administered to the patient by the systemic route, for instance intravenously, or may be administered locally to the tumour site. The local administration may be intraportal, intraarterial or intratumoral. In the case of intravenous administration, the PPIs are preferably administered in the form of the PEGylated liposomes according to the invention. The PPIs or liposomes containing PPI may be administered before, during or after the thermal ablation procedure, by methods easily determinable by the skilled person. For therapeutic applications, the PPIs and their liposomal forms according to the invention are formulated as conventional pharmaceutical compositions comprising PPIs or their liposomal forms mixed with one or more excipients approved for pharmaceutical use.

The PEGylated liposomes loaded with omeprazole in acidic solution according to the present invention have special pharmacological features that allow drug deposition at a high concentration and in the active form selectively at tumour sites, by-passing entrapment and functional activation which, for PPIs administered as such in free form, are limited to the acidic gastric environment.

Encapsulation in PEGylated liposomes ("stealth" liposomes) allows exploitation of the increased endothelial permeability which is a tumour characteristic. Such permeability, which is strongly enhanced by the local heating that takes place during hyperthermic tumour ablation, allows the extravasation of long-circulating liposomes and their concentration in the interstitial area of the tumour (*enhanced permeation and retention effect*), where the pharmacological payload is released as a consequence of normal degradation of the phospholipid carrier. Furthermore, the use of the PEGylated liposomes loaded with omeprazole in acidic solution according to the present invention allows prior transformation of the prodrug into its active form, regardless of the extracellular pH of the solid tumour in vivo, which can vary from acidic to slightly alkaline.

The invention is now further illustrated by the following examples

**Example 1**

***Preparation of PEGylated liposomes (LUVs) loaded with omeprazole in acidic solution***

The entrapment of omeprazole in PEGylated LUV (large unilamellar vesicle) liposomes has been obtained with the lipid film hydration method followed by extrusion through a membrane. In detail, the liposomes were prepared so as to obtain an acidic core. The acid chosen was lactic acid (Fluka, Milan, Italy) which, being a cell metabolite, exhibits a high level of biocompatibility. The lipid composition was hydrogenated soy phosphatidylcholine (HSPC, Lipoid, Milan, Italy), PEGylated distearoylphosphatidylethanolamine (DSPE-mPEG2000, Lipoid, Milan, Italy) and cholesterol (Chol, Fluka, Milan, Italy). The lipid mixture employed was HSPC:DSPE-mPEG2000:Chol 60:20:20 (% w/w).

In order to obtain multilamellar vesicles (MLVs), the lipids and omeprazole (Sigma-Aldrich, Milan, Italy) were dissolved in chloroform in a 95:5 (% w/w) ratio; the organic solution was evaporated under nitrogen stream to form a thin lipid layer; and the film was hydrated at 60°C with a 50 mM lactate buffer pH 5.0.

The resulting dispersion was cooled and left to stand overnight at 4°C. The MLVs were then extruded through 200 and 100 nm membrane cut-off polycarbonate filters placed in series at 60°C (LiposoFast Polycarbonate Membrane for Liposofast Liposome Factory, Sigma-Aldrich, Milan, Italy). Nine extrusion cycles were performed. After being left to stand at 4°C, the LUVs obtained were ultracentrifuged at 90000 rpm for 1 hour at 4°C (Optima TL 100000 rpm, Beckman, Milan, Italy) and the acidic supernatant containing non-encapsulated omeprazole was removed by two washing cycles and subsequent centrifugation with saline solution. The pellet was suspended in saline solution and stored at 4°C.

PEGylated LUVs only loaded with lactate buffer pH 5.0 solution (LUVs with acidic core) were prepared by the same procedure.

All preparations were performed under aseptic conditions.

The liposomes obtained were characterized as described hereafter.

*Omeprazole content.* The LUV liposomes were characterized to determine omeprazole content. Omeprazole extraction from the LUV suspension was obtained by ultracentrifugation of an aliquot of the suspension (90000 rpm, 1 hour, 4°C). After removal of the supernatant and freeze-drying (Benchtop 2K freeze-dryer - Virtis, New York), the pellet was solubilized in an established volume of the $CH_3OH/CHCl_3/DMSO$ 1:5:94 (% v/v/v) solvent mixture. Omeprazole content was obtained by UV-vis spectrophotometric analysis, at $\lambda_{max}$ = 301 nm, using an Agilent 8453 spectrophotometer (Agilent, Milan, Italy). Calibration was performed in DMSO in the 2.5-20 $\mu$g/mL concentration range (r2=0.99854). The amount of omeprazole in the liposomes was expressed as % drug content (DC):

$$DC\,(\%) = \frac{mg\ omeprazole}{mg\ of\ liposomes} \cdot 100 \qquad (1)$$

and the % encapsulation efficiency (EE) as:

$$EE\,(\%) = \frac{Amount\ of\ omeprazole}{Total\ amount\ of\ omeprazole\ added} \cdot 100 \qquad (2)$$

The measurements were performed in triplicate and the results expressed as mean $\pm$ S.D. The results are reported in Table 1.

Table 1 - Characteristics of PEGylated LUVs prepared with HSPC:DSPE-MPEG2000-Chol (60:20:20 %w/w) by the lipid film hydration and extrusion method.

| Batch | Hydration volume | Process | DC (%w/w) $\pm$S.D. | EE (%)$\pm$S.D | MHD (nm) | P1 |
|---|---|---|---|---|---|---|
| #1 | 8 mL | Not extruded | 3.4$\pm$0.2 | 16.9$\pm$1.6 | 104.3 | 0.213 |
| | | Extruded | 0.6$\pm$0.3 | | | |
| #2 | 7 mL | Not extruded | 4.0$\pm$0.4 | 7.2$\pm$0.4 | 114.7 | 0.179 |
| | | Extruded | 0.3$\pm$0.1 | | | |
| #3 | 6.5 mL | Not extruded | 4.0$\pm$0.5 | 26.8$\pm$1.2 | 100.9 | 0.259 |
| | | Extruded | 1.1$\pm$0.3 | | | |
| #4 | 6 mL | Not extruded | 5.6$\pm$0.4 | 23.6$\pm$1.5 | 112.3 | 0.127 |
| | | Extruded | 1.3$\pm$0.2 | | | |
| #5 | 5 mL | Not extruded | 4.3$\pm$0.2 | 55.3$\pm$2.4 | 118.8 | 0.115 |
| | | Extruded | 2.4$\pm$0.3 | | | |

Evaluation of the omeprazole content and encapsulation efficiency showed that the volume of the aqueous solution used for the hydration of the film is critical. In general, the reduction in the hydration volume produced an increase in both DC and EE after extrusion. The optimal value was 6 mL, since smaller volumes, although they produced greater drug encapsulation, caused precipitation of omeprazole, making the extrusion process very difficult, with considerable loss of material. The omeprazole content ranged between 1-2% w/w. As shown in Table 1, the extrusion process caused a drastic loss of drug content, an entirely predictable phenomenon due to the nature of the process, which involves reorganization of the MLVs to LUVs by fusion of the bilayer into one membrane at temperatures at least 10°C above the transition temperature of the lipids.

*Size and morphology analyses.* Size distribution analysis was performed with dynamic light scattering (DLS) measurements using a Nicomp 380 autocorrelator (PSS Inc., Santa Barbara, USA) equipped with a 35 mW Argon ion Coherent Innova 70-3 laser (Laser Innovation, Moorpark, USA) at $\lambda$ = 658 nm and APD detector. All samples were suitably diluted in saline solution before each analysis. The mean size and size distribution width of the LUVs were expressed as mean hydrodynamic diameter (MHD) and polydispersity index (PI) respectively. The results are reported in Table 1. The PEGylated LUVs loaded with omeprazole had a mean diameter of around 100 nm and a small polydispersity index. This is the result of the extrusion process obtained with nine cycles through membranes with a porosity of 200 and 100 nm placed in series.

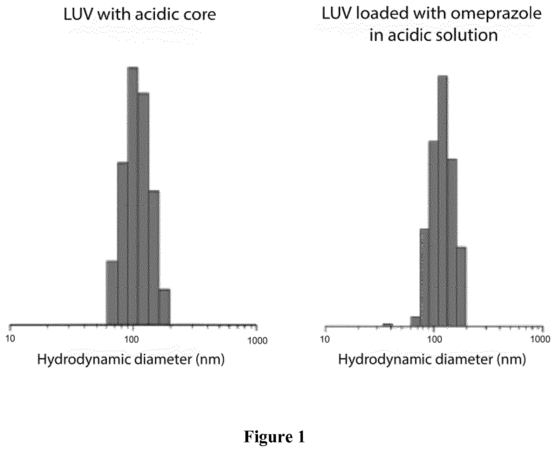

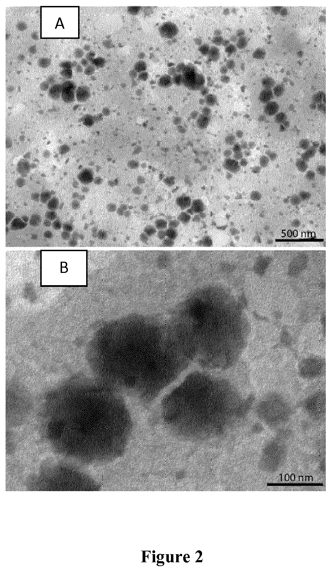

Liposome morphology was observed with a Philips EM 400T (Philips, Eindhoven, NL) transmission electron microscope (TEM). Samples were prepared by depositing a drop of suspension on Formvar-coated TEM copper grids pre-treated with a polylysine solution. After liposome adhesion, excess water was removed using filter paper, and the sample was stained with 1% (w/v) phosphotungstic acid for 2 minutes and analysed. Figure 1 shows two examples of size distributions of LUVs loaded and not loaded with the drug obtained with the DLS measurements. The extreme homogeneity of the vesicles demonstrates the effectiveness of the extrusion process. Furthermore, there are no significant differences between the profiles of the loaded and non-loaded LUVs apart from a minimal average increase in size (from 100 to 120 nm). This shows that loading with the drug does not induce significant changes in the structure of the vesicles. The morphological analysis of the loaded LUVs confirmed the presence of homogeneous vesicles with a regular shape and dimensions, in agreement with the light scattering results (Figure 2).

### Example 2

#### *Preparation of acidic omeprazole solution*

An acidic omeprazole solution was prepared to allow biological comparison of the effect of omeprazole in acidic solution entrapped in PEGylated LUVs with that of the free drug (non-liposomal). The solution was obtained using 50 mM lactate buffer pH 5.0, analogous to the solution entrapped in LUVs of example 1. The drug was dissolved by sonication and gentle heating. The acidic solution of omeprazole was sterilized by filtration through 0.2 $\mu$m sterile filters (Millipore, Milan, Italy).

### Example 3

#### *In vitro studies on HT-29 (human colon adenocarcinoma) cells*

The thermosensitizing or killing effect of omeprazole in acidic solution and that of liposome formulations was tested on human colon adenocarcinoma HT-29 cell line (Istituto Zooprofilattico Ubertini, Brescia, Italy). The cells were incubated under standard conditions: 5% $CO_2$, 37°C, in McCOY'S 5A medium (Sigma-Aldrich, Milan, Italy) at pH 7.4 with sodium bicarbonate, antibiotics and antifungals (penicillin 100 U/mL, streptomycin 100 U/mL, amphotericin B 0.25 $\mu$g/mL) and 10% foetal bovine serum. A trypsin solution with EDTA was employed to detach the cells from the culture plates.

The experimental protocol is reported in the following scheme:

Plating of HT-29 cells (250000/well with 5 mL medium, initial pH = 7.4 buffered)

↓ 72 h at 37°C

$T_0$: Replacement of the medium with unbuffered medium (without sodium bicarbonate) and drug addition *

↓ 24 h at 37°C

Thermal treatment: 2 hours at 45°C (controls at 37°C)

↓ 24 h at 37°C

Evaluation in triplicate of: final pH, total number of cells (living + dead), % viability^.

Endpoint: total number of living cells

---

* A. Controls (not treated); B. Omeprazole acidic solution (pH 5.0), 200 μL/well (160 μM); C. PEGylated LUVs loaded with acidic solution, 200 μL/well; D. PEGylated LUVs loaded with omeprazole acidic solution, 200 μL/well (160 μM).

∧ Staining with ethidium bromide and fluorescein diacetate.

In detail:

1)-HT-29 cells were seeded in 6-well culture plates (Falcon) with a 10 cm$^2$ area at a 250,000 cell/well concentration and 5 mL medium as indicated above.

2)-After 72 h plating, the culture medium, with initial buffered pH=7.4, was replaced with unbuffered medium (without sodium bicarbonate), and 12 wells were maintained at 37°C, while 12 wells were destined for thermal treatment. In each group, three wells were treated with omeprazole in acidic solution, three with LUVs with acidic core and three with LUVs loaded with omeprazole in acidic solution, according to the concentrations reported in the previous scheme. Three wells in each group did not receive any pharmacological treatment (controls).

3)-After 24 h of incubation at 37°C with omeprazole and with the liposomal formulations, without replacing the medium, one of the 12-well group was subjected to thermal treatment for 2 h at 45°C in a water bath, while the other was maintained at 37°C as a control. At the end of the thermal treatment the cells were brought back into the incubator at 37°C.

4)-After 24 h, the following parameters were evaluated for all the wells:

*Total number of cells (living and dead).* After detachment of the cell monolayer with trypsin and EDTA, the total number of cells (living + dead) in each well was determined using a Countess cell counting machine (Invitrogen, Milan, Italy).

*Cell viability (%).* Cell viability was evaluated by incubation of a detached cell suspension with ethidium bromide and fluorescein diacetate (Sigma-Aldrich, Milan, Italy). The cells were analysed with a fluorescent optical microscope (Olympus BX-41, Tokyo, Japan) using the filter for fluorescein. The images were recorded with an F-view digital camera (Olympus) and processed with F-view software (Olympus). With this method, the living cells are stained green and the dead cells red, as fluorescein diacetate is internalized by living cells and hydrolysed by cytoplasm esterases into the corresponding fluorochrome, fluorescein, which emits an intense green fluorescence, whereas ethidium bromide only penetrates cells with altered membranes, binding to nucleic acids and producing a red fluorescence.

The living and dead cells were counted by a semi-manual method. A minimum of three images per well were acquired in distinct areas, representative of the corresponding cell suspension. For the group maintained at 37°C, an average of 2,500 cells was counted for each treatment type (constituted by three wells representing the single treatment performed in triplicate). Since the number of cells decreases noticeably for the wells treated at 45°C for 2 h, the number of recorded images was increased (from 9 to 16), and 2,700 to 3,500 cells were counted for each treatment type (constituted by three wells representing the single treatment performed in triplicate). Living cell (green) and dead cell (red) counts were acquired by marking each cell and recording the resulting cell number with Image J software.

The percentage viability of the cells counted for each sample was calculated as follows:

$$\text{Viability (\%)} = \frac{\text{No. of living cells}}{\text{No. of living cells} + \text{No. of dead cells}} \cdot 100 \qquad (3)$$

*Total number of living cells.* The endpoint of the study was the total number of tumour cells surviving the different treatments. This value was calculated for each well by applying the viability percentage obtained by semi-manual counting to the total number of cells measured by the cell-counting machine.

*Statistical analysis.* The data were subjected to statistical analysis with the paired Student's t-test and one-way ANOVA (SigmaPlot 12.0, Systat Software Inc.) with a 95% significance level. Bonferroni's post-hoc method was used to identify differences within groups. All values were expressed as mean $\pm$ S.D..

The following results were obtained.

Cell survival at 37°C

The HT-29 cells maintained at 37°C as a control proliferated over five days after plating ($0.25 \times 10^6$/well), reaching a population of over $2 \times 10^6$ cells/well, with an average viability of about 94%, equal to $2.0 \times 10^6$ living cells (Table 2).

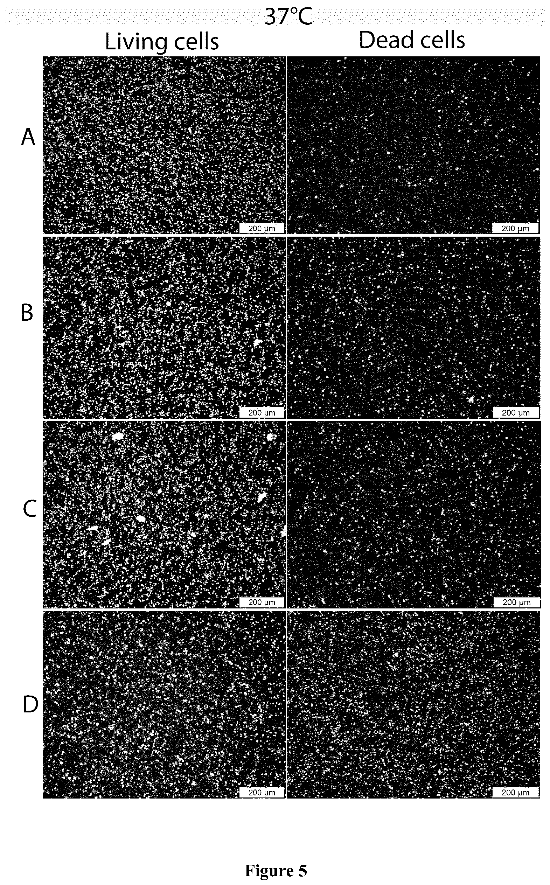

In line with the data reported in the literature, which indicate that under normal thermal conditions, omeprazole exerts a cytotoxic effect against various tumour cell lines in vitro, our study showed that treatment with omeprazole (in acidic solution) reduces the population and the percentage viability of colon adenocarcinoma cell cultures at 37°C (Table 2). The number of tumour cells surviving after 48 h of incubation with the drug was 25% lower than in the untreated control (Table 2). This difference is statistically significant (Table 3: B. vs. A. at 37°C). However, the decrease in cell survival was much higher (-64%) when the cell culture was incubated with omeprazole encapsulated in LUVs (Table 2; Figure 5D). As reported in Table 3, the difference between the two formulations was highly significant (D. vs. B. at 37°C). Even LUVs with only an acidic core reduced cell survival (Table 2), but to a significantly lower extent than LUVs loaded with omeprazole in acidic solution (Table 3: D. vs. C. at 37°C; Figure 5).

**Table 2** - Total number of cells (living and dead), % viability, and number of living cells after pharmacological treatment at 37°C and after exposure to 45°C for 2 h. **A.** Untreated controls; **B.** Omeprazole in acidic solution; **C.** LUVs with acidic core; **D.** LUVs loaded with omeprazole in acidic solution.

| Treatment | 37°C | | | 45°C for 2h | | |
|---|---|---|---|---|---|---|
| | Total cells ($\times 10^6$) | Viability (%) | Living cells ($\times 10^6$) | Total cells ($\times 10^6$) | Viability (%) | Living cells ($\times 10^6$) |
| **A** | $2.13 \pm 0.12$ | $94.1 \pm 0.5$ | $2.00 \pm 0.12$ | $1.09 \pm 0.18$ | $64.4 \pm 6.3$ | $0.68 \pm 0.07$ |
| **B** | $1.83 \pm 0.25$ | $83.3 \pm 2.4$ | $1.53 \pm 0.22$ | $0.66 \pm 0.27$ | $61.5 \pm 4.4$ | $0.40 \pm 0.10$ |
| **C** | $1.40 \pm 0.27$ | $83.6 \pm 0.8$ | $1.17 \pm 0.22$ | $0.66 \pm 0.15$ | $50.4 \pm 4.0$ | $0.35 \pm 0.03$ |
| **D** | $1.27 \pm 0.12$ | $56.4 \pm 6.5$ | $0.72 \pm 0.15$ | $0.73 \pm 0.01$ | $18.3 \pm 2.5$ | $0.13 \pm 0.02$ |

**Table 3** - Statistical significance (ANOVA, $\alpha$ = 0.05) of differences in cell survival at 37°C and after exposure to 45°C for 2 h according to the pharmacological treatments (*p* value): **A.** Untreated controls; **B.** Omeprazole in acidic solution; **C.** LUVs with acidic core; **D.** LUVs loaded with omeprazole in acidic solution.

| | 37°C | | | | 45°C for 2h | | | |
|---|---|---|---|---|---|---|---|---|
| | A | B | C | D | A | B | C | D |
| **A** | ---- | 0.008 | <0.001 | <0.001 | ---- | <0.001 | <0.001 | <0.001 |
| **B** | ---- | ---- | 0.035 | <0.001 | ---- | ---- | 0.065 | <0.001 |
| **C** | ---- | ---- | ---- | 0.043 | ---- | ---- | ---- | <0.001 |

(continued)

|  | 37°C | | | | 45°C for 2h | | | |
|---|---|---|---|---|---|---|---|---|
|  | A | B | C | D | A | B | C | D |
| D | ---- | ---- | ---- | ---- | ---- | ---- | ---- | ---- |

Cell survival 24 h after thermal treatment (45°C for 2 h)

Although heating at 45°C for 2 h significantly reduced cell survival compared to the control at 37°C (Table 2; Table 4: A. at 37°C vs A. at 45°C), about a third of the tumour cells survived 24 h after the thermal shock. This model of suboptimal thermal necrosis allowed the thermosensitizing effect of drug treatments to be tested, as required by the purpose of the study.

As reported in Table 2, pre-treatment with omeprazole in acidic solution, with LUVs with acidic core, and with LUVs loaded with omeprazole in acidic solution, all reduced, with variable success, the number of cells and the percentage viability of the cell cultures 24 h after the heat shock.

**Table 4** – Statistical significance (ANOVA, $\alpha = 0.05$) of cell survival differences between drug treatments at 37°C and the same treatment after exposure to 45°C for 2 h ($p$ value). **A.** Untreated controls; **B.** Omeprazole in acidic solution; **C.** LUVs with acidic core; D. LUVs loaded with omeprazole in acidic solution.

| 37°C / 45°C | A | B | C | D |
|---|---|---|---|---|
| A | <0.001 | ---- | ---- | ---- |
| B | ---- | <0.001 | ---- | ---- |
| C | ---- | ---- | <0.001 | ---- |
| D | ---- | ---- | ---- | <0.001 |

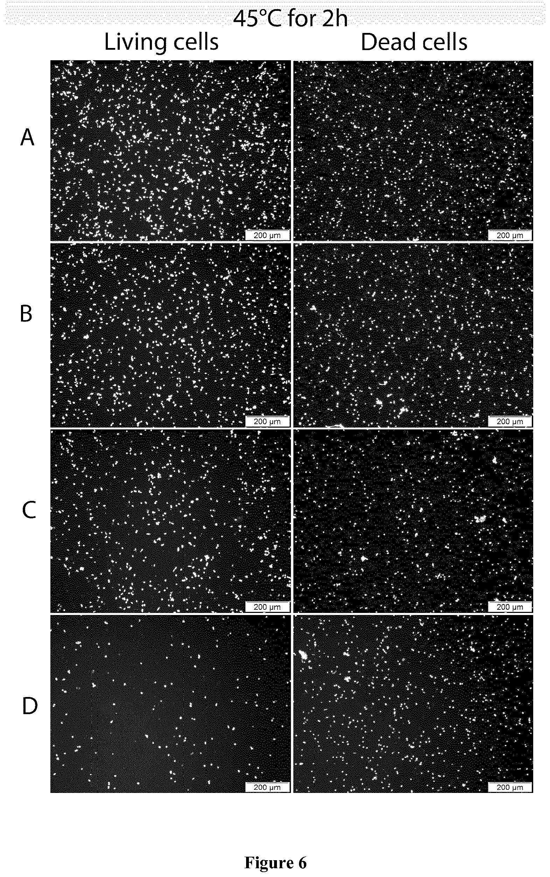

Compared to heating only, pre-treatment of the cell cultures with omeprazole in acidic solution decreased the number of living cells by 41% (Table 2), a highly significant difference (Table 3: B. vs. A. at 45°C). The reduction in survival rose to 49% when the cell cultures were incubated with the LUVs with acidic core before heat treatment (Table 2), but the difference was not statistically significant compared with the cells treated with omeprazole in acidic solution (Table 3: C. vs B. at 45°C). As shown in Figure 6, the combination of heat treatment with the administration of LUVs loaded with omeprazole in acidic solution produced a dramatic 81% reduction in the number of surviving cells compared with heating only (Table 2). This survival reduction is significantly higher than that obtained with either omeprazole in acidic solution (Table 3: D. vs B. at 45°C) or LUVs loaded with an acidic solution (Table 3: D. vs. C. at 45°C). With reference to the final number of viable control cells at 37°C ($2.0 \times 10^6$), only 6.5% of the tumour cells survived the combined treatment with hyperthermia and LUVs loaded with omeprazole in acidic solution (Table 2; compare Figures 5 and 6).

**Claims**

1.   A proton pump inhibitor encapsulated in a PEGylated liposome wherein the inhibitor is dissolved in a solution at a pH ranging between 5 and 7.4 for use in the treatment of tumours in combination with a tumour thermoablation method.

2.   A proton pump inhibitor for use according to claim 1, wherein the proton pump inhibitor is selected from the group consisting of omeprazole, esomeprazole, lansoprazole, pantoprazole and rabeprazole.

3. A proton pump inhibitor for use according to claim 2, wherein the proton pump inhibitor is omeprazole.

4. A proton pump inhibitor for use according to anyone of the preceding claims , wherein the liposome is thermosensitive.

5. A proton pump inhibitor encapsulated in a PEGylated liposome wherein the inhibitor is dissolved in a solution at a pH ranging between 5 and 7.4, wherein the proton pump inhibitor is selected from the group consisting of omeprazole, esomeprazole, lansoprazole and pantoprazole .

6. A proton pump inhibitor encapsulated in a PEGylated liposome according to claim 5 wherein the proton pump inhibitor is selected from the group consisting of omeprazole, esomeprazole and lansoprazole.

7. The proton pump inhibitor encapsulated in PEGylated liposome according to claim 5, wherein the proton pump inhibitor is omeprazole.

8. The proton pump inhibitor encapsulated in a PEGylated liposome according to any one of claims 5 to 7 wherein the liposome comprises hydrogenated phosphatidylcholine, distearoyl phosphatidylethanolamine pegylated with polyethylene glycol 2000 and cholesterol in a weight ratio of 60:20:20, wherein the weight ratio between lipids and inhibitor is 95:5.

9. The proton pump inhibitor encapsulated in a PEGylated liposome according to any one of claims 5 to 8 for use as adjuvant in treatment of tumours by hyperthermic thermal tumour ablation procedures.

10. A pharmaceutical formulation comprising a proton pump inhibitor encapsulated in a PEGylated liposome according to any one of claims 5 to 8 and a suitable carrier and/or excipient.

**Patentansprüche**

1. Protonenpumpen-Inhibitor, der in ein PEGyliertes Liposom eingekapselt ist, wobei der Inhibitor in einer Lösung mit einem pH im Bereich zwischen 5 und 7,4 gelöst ist, zur Verwendung in der Behandlung von Tumoren in Kombination mit einem Tumorthermoablations-Verfahren.

2. Protonenpumpen-Inhibitor zur Verwendung gemäß Anspruch 1, wobei der Protonenpumpen-Inhibitor aus der Gruppe, bestehend aus Omeprazol, Esomeprazol, Lansoprazol, Pantoprazol und Rabeprazol, ausgewählt ist.

3. Protonenpumpen-Inhibitor zur Verwendung gemäß Anspruch 2, wobei der Protonenpumpen-Inhibitor Omeprazol ist.

4. Protonenpumpen-inhibitor zur Verwendung gemäß einem der vorangehenden Ansprüche, wobei das Liposom wärmeempfindlich ist.

5. Protonenpumpen-Inhibitor, der in ein PEGyliertes Liposom eingekapselt ist, wobei der Inhibitor in einer Lösung mit einem pH zwischen 5 und 7,4 gelöst ist, wobei der Protonenpumpen-Inhibitor aus der Gruppe, bestehend aus Omeprazol, Esomeprazol, Lansoprazol und Pantoprazol, ausgewählt ist.

6. Protonenpumpen-Inhibitor, der in ein PEGyliertes Liposom eingekapselt ist, gemäß Anspruch 5, wobei der Protonenpumpen-inhibitor aus der Gruppe, bestehend aus Omeprazol, Esomeprazol und Lansoprazol, ausgewählt ist.

7. Protonenpumpen-Inhibitor, der in ein PEGyliertes Liposom eingekapselt ist, gemäß Anspruch 5, wobei der Protonenpumpen-Inhibitor Omeprazol ist.

8. Protonenpumpen-Inhibitor, der in ein PEGyliertes Liposom eingekapselt ist, gemäß einem der Ansprüche 5 bis 7, wobei das Liposom hydriertes Phosphatidylcholin, distearoylphosphatidylethanolamin, pegyliert mit Polyethylenglycol 2000, und cholesterin in einem Gewichtsverhältnis von 60:20:20 umfasst, wobei das Gewichtsverhältnis zwischen Lipiden und Inhibitor 95:5 ist.

9. Protonenpumpen-Inhibitor, der in ein PEGyliertes Liposom eingekapselt ist, gemäß einem der Ansprüche 5 bis 8 zur Verwendung als Adjuvans in der Behandlung von Tumoren durch hypertherme Tumor-Wärmeablations-Verfahren.

**10.** Pharmazeutische Formulierung, umfassend einen Protonenpumpen-Inhibitor, der in ein PEGyliertes Liposom eingekapselt ist, gemäß einem der Ansprüche 5 bis 8 und einen geeigneten Träger und/oder Hilfsstoff.

**Revendications**

**1.** Inhibiteur de pompe à protons encapsulé dans un liposome pégylé dans lequel l'inhibiteur est dissous dans une solution à un pH se situant dans la plage comprise entre 5 et 7,4 pour une utilisation dans le traitement de tumeurs en combinaison avec un procédé de thermoablation de tumeur.

**2.** Inhibiteur de pompe à protons pour une utilisation selon la revendication 1, dans lequel l'inhibiteur de pompe à protons est choisi dans le groupe constitué par l'oméprazole, l'ésoméprazole, le lansoprazole, le pantoprazole et le rabéprazole.

**3.** Inhibiteur de pompe à protons pour une utilisation selon la revendication 2, dans lequel l'inhibiteur de pompe à protons est l'oméprazole.

**4.** Inhibiteur de pompe à protons pour une utilisation selon l'une quelconque des revendications précédentes, dans lequel le liposome est thermosensible.

**5.** Inhibiteur de pompe à protons encapsulé dans un liposome pégylé dans lequel l'inhibiteur est dissous dans une solution à un pH se situant dans la plage comprise entre 5 et 7,4, l'inhibiteur de pompe à protons étant choisi dans le groupe constitué par l'oméprazole, l'ésoméprazole, le lansoprazole et le pantoprazole.

**6.** Inhibiteur de pompe à protons encapsulé dans un liposome pégylé selon la revendication 5 dans lequel l'inhibiteur de pompe à protons est choisi dans le groupe constitué par l'oméprazole, l'ésoméprazole et le lansoprazole.

**7.** Inhibiteur de pompe à protons encapsulé dans un liposome pégylé selon la revendication 5, dans lequel l'inhibiteur de pompe à protons est l'oméprazole.

**8.** Inhibiteur de pompe à protons encapsulé dans un liposome pégylé selon l'une quelconque des revendications 5 à 7 dans lequel le liposome comprend de la phosphatidylcholine hydrogénée, de la phosphatidyléthanolamine de distéaroyle pégylée avec du polyéthylène glycol 2000 et du cholestérol dans un rapport en poids de 60:20:20, le rapport en poids entre les lipides et l'inhibiteur étant de 95:5.

**9.** Inhibiteur de pompe à protons encapsulé dans un liposome pégylé selon l'une quelconque des revendications 5 à 8 pour une utilisation comme adjuvant dans le traitement de tumeurs par des procédures d'ablation de tumeur thermique hyperthermiques.

**10.** Formulation pharmaceutique comprenant un inhibiteur de pompe à protons encapsulé dans un liposome pégylé selon l'une quelconque des revendications 5 à 8 et un véhicule et/ou excipient approprié.

LUV with acidic core       LUV loaded with omeprazole in acidic solution

Hydrodynamic diameter (nm)       Hydrodynamic diameter (nm)

**Figure 1**

**Figure 2**

**Figure 3**

Figure 4

**Figure 5**

45°C for 2h
Living cells          Dead cells

A
B
C
D

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5013556 A

### Non-patent literature cited in the description

- **AHMED M.** *Chem Phys Lipids,* 2012, vol. 165, 424-37
- **CHU KF.** *Nat Rev Cancer,* 2014, vol. 14, 199-208
- **PAIN D.** *J. Biosci,* 1984, vol. 6, 811-6
- **NING S.** *Soft Matter,* 2011, vol. 7, 9394-401
- **KONING GA.** *Pharm Res,* 2010, vol. 27, 1750-4
- **LANDON CD.** *Open Nanomed J,* 2011, vol. 3, 38-64
- **MANZOOR AA.** *Cancer Res,* 2012, vol. 72, 5566-75
- **KNEIDL B.** *Int J Nanomedicine,* 2014, vol. 9, 4387-98
- **LENCIONI R.** *Presentation at the European Conference on Interventional Oncology (ECIO), Budapest (Hungary),* 19 June 2013
- **POON RT.** *Presentation at the World Conference on Interventional Oncology (WCIO),* 16 May 2013
- **FINN RS.** *Presentation at the 8th International Liver Cancer Association (ILCA) Annual Conference, Kyoto (Japan),* 05 September 2014
- **BARONZIO GF.** Hyperthermia in cancer treatment. Landes-Springer Publishers, 2006, 67-91
- **SONG CW.** Cancer drug resistance Teicher BA (ed). Humana Press, 2006, 21-42
- **JONES EL.** Principles and practice of radiation oncology. Lippincott Williams & Wilkins, 2008, 637-68
- **VIGLIANTI BL et al.** Holland-Frei cancer medicine 8th ed. People's Medical Publishing House-USA, 2010, 528-40
- **HELANDER HF.** *Scand J Gastroenterol,* 1985, vol. 20 (108), 95-104
- **OLBE L.** *Nat Rev Drug Discov,* 2003, vol. 2, 132-9
- **DE MILITO A.** *Curr Pharm Des,* 2012, vol. 18, 1395-406
- **SPUGNINI EP.** *J Exp Clin Cancer Res,* 2010, vol. 29, 44
- **FAIS S.** *J Intern Med,* 2010, vol. 267, 515-25
- **PARKS SK.** *Nat Rev Cancer,* 2013, vol. 13, 611-23
- **FAIS S.** *Cancer Metastasis Rev,* 2014, vol. 33, 1095-108
- **MATUSZCAK C.** *Can Cell Microenviron,* 2015, vol. 2, e667
- **WALSH M.** *J Exp Clin Cancer Res,* 2015, vol. 34, 93
- **WANG BY.** *J Exp Clin Cancer Res,* 2015, vol. 34, 85